# EUROPEAN PATENT SPECIFICATION

(11) **EP 1 174 085 B1**
(45) Date of publication and mention of the grant of the patent: **09.07.2003**
(21) Application number: 01126655.8
(22) Date of filing: 03.03.1998
(51) Int. Cl.: A61B 5/15, B65D 41/04

(54) **Assembly for collecting blood or other body fluids**
Vorrichtung zum Sammeln von Blut oder anderen Körperflüssigkeiten
Dispositif pour collecter du sang ou d'autres fluides corporels

(30) Priority: 26.03.1997 US 42062 P
(43) Date of publication of application: 23.01.2002
(62) Divisional of application: 98103704.7
(73) Proprietor: Becton Dickinson and Company, Franklin Lakes, New Jersey 07417-1880 (US)
(72) Inventor: Newby, Mark C., Tuxedo, New-York 10987 (US); Miller, Henry F., Clifton, New Jersey (US); Schleiffer, Keith E., Gahanna, Ohio 43230 (US)
(74) Representative: Selting, Günther, Dipl.-Ing.

(56) References cited:
- EP-A- 0 513 901
- US-A- 4 967 919

## Description

The field of the invention relates to assemblies for collecting blood or other body fluids, the assemblies being of the type including a container having a closure assembly pierceable by a needle or cannula.

Blood collection tubes are commonly used by doctors, nurses and others persons to draw a sample of a body fluid from a patient or to receive a fluid sample from another vessel. Such tubes are ordinarily evacuated, and include a pierceable closure. During one typical use of a blood collection tube, one end of a double-ended needle assembly is used to pierce a vein. The evacuated blood collection tube is then urged towards the second end of the double-ended needle assembly until its closure is pierced. Blood is thereby drawn into the tube.

The double-ended needle assembly is ordinarily mounted to a holder having a tubular body. The blood collection tube is inserted within the tubular body in order to engage the second end of the double-ended needle assembly.

The maintenance of a vacuum within the blood collection tube is important. Blood collection tubes may be stored for considerable lengths of time before they are used. Loss of vacuum during storage or at any time can render the collection tube less effective or useless.

A number of design of blood collection tubes and their closures have been the subjects of patents and other disclosures. British Patent 1 388 494 and U.S. Patent No. 4,327,746 both disclose closures for such tubes having sealing membranes positioned between a cap and the open end of the collection tube. U.S. Patent No. 5,061,263 discloses several closure designs, each including a cap, a gas barrier member and a re-sealable member.

An assembly for collecting blood or other body liquids, according to the precharacterizing part of claim 1, is disclosed in EP 0 513 901 A1. This blood collecting assembly comprises a cylindrical container and a closure assembly for closing the open end of the container. The closure assembly is a plastic cap defining a chamber for receiving an inserted stopper. The closure assembly can be pressed on the open end of the container so that the stopper will be inserted into the open end whereas the plastic cap surrounds the top portion of the container. The cap has an opening for permitting access to the interior of the container. The container has on its open end a bead for cooperating with an undercut of the cap so that the cap will snap into its position on the container.

U.S. Patent No. 4,150,666, 4,967,919, 4,991,601, 5,326,534, 5,494,170 and International Publication No. WO 81/01238 disclose various blood collection tubes and/or holder for such tubes. The '170 Patent discloses a cap having flexible tabs which engage the wall of the holder, thereby maintaining the blood collection tube within the holder.

It is an object of the present invention to provide a body fluid collection assembly which provides a superior interference fit when the container is inserted into a tube holder.

The present invention is defined by Claim 1.

Further, preferred embodiments are disclosed in dependent claims 2 to 6.

The cap of the invention includes a deflectable portion at the top end thereof. The deflectable portion should be designed to engage a planar surface if the container is placed horizontally on such a surface. This allows the deflectable portion to function as a shock absorbing system which will help preserve the bond between the gas barrier and the container should the container be dropped. It also provides a superior interference fit when the container is inserted within a tube holder.
Fig. 1 is a perspective view of an assembly for collecting body fluids and a tube holder capable of receiving a portion of the assembly;
Fig. 2 is a top perspective view showing the assembly having an end portion positioned within the tube holder;
Fig. 3 is a sectional elevation view showing the assembly for collecting body fluids and the tube holder;
Fig. 4 is a sectional elevation view showing the assembly positioned within the tube holder;
Fig. 5 is an enlarged, sectional view showing the assembly fully inserted within the tube holder;
Fig. 6 is an exploded, perspective view of the assembly for collecting body fluids;

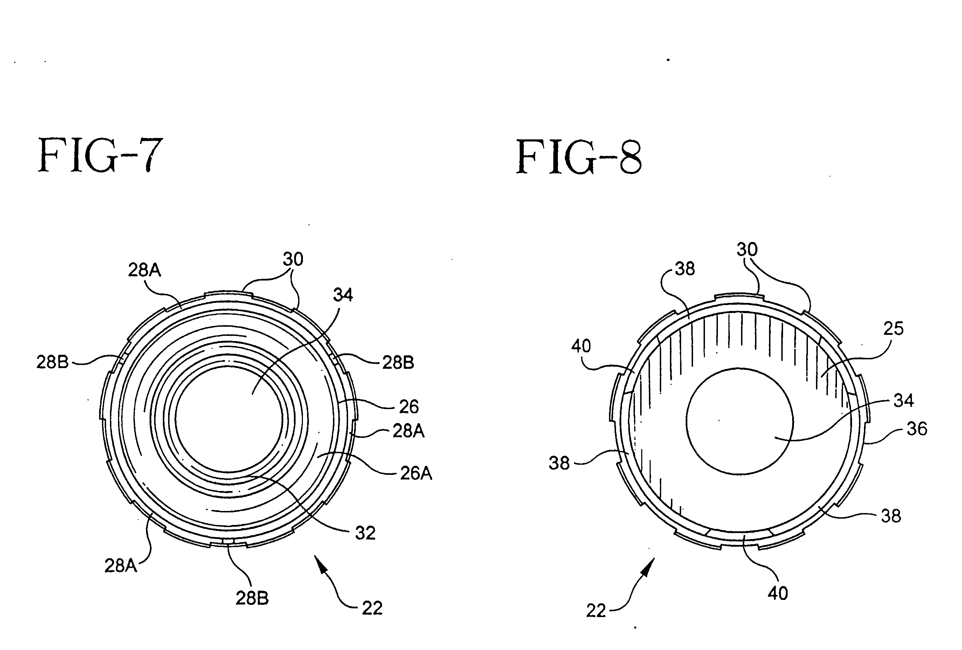

Fig. 7 is a top plan view of a cap of the assembly for collecting body fluids;
Fig. 8 is a bottom plan view thereof, and
Fig. 9 is a bottom perspective view thereof.

### DETAILED DESCRIPTION OF THE INVENTION

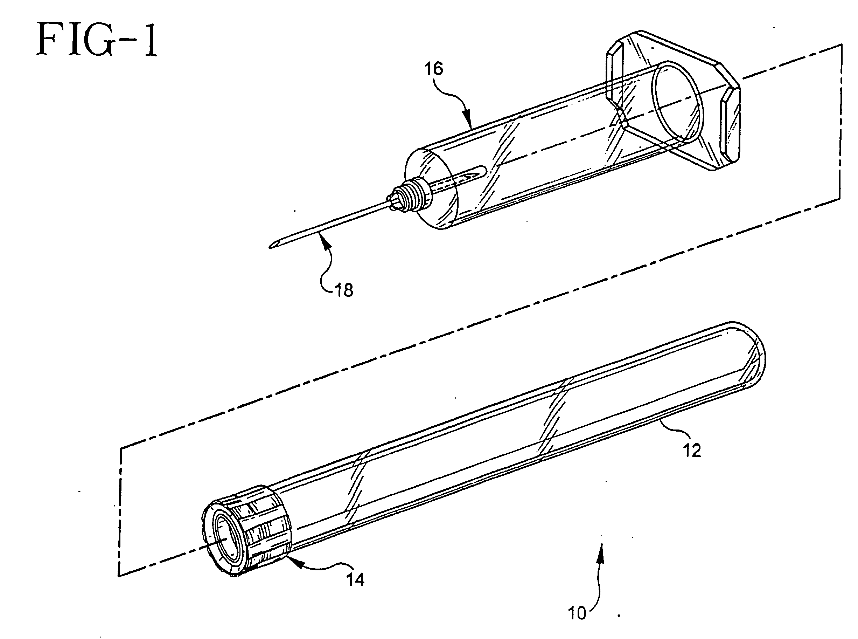

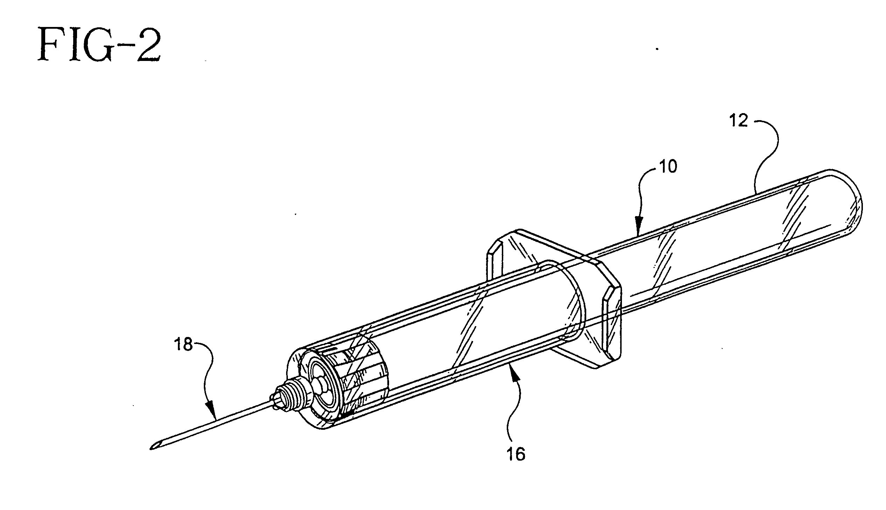

An assembly 10 for collecting blood or other body fluids is provided by the invention. Referring to Figs. 1-6, the assembly includes a generally cylindrical container 12 having a closed end and an open end, and a closure assembly 14 which can be removably mounted to the open end of the container. As shown in Figs. 1-5, the container may be employed in conjunction with a holder 16 to which a double-ended needle assembly 18 is mounted.

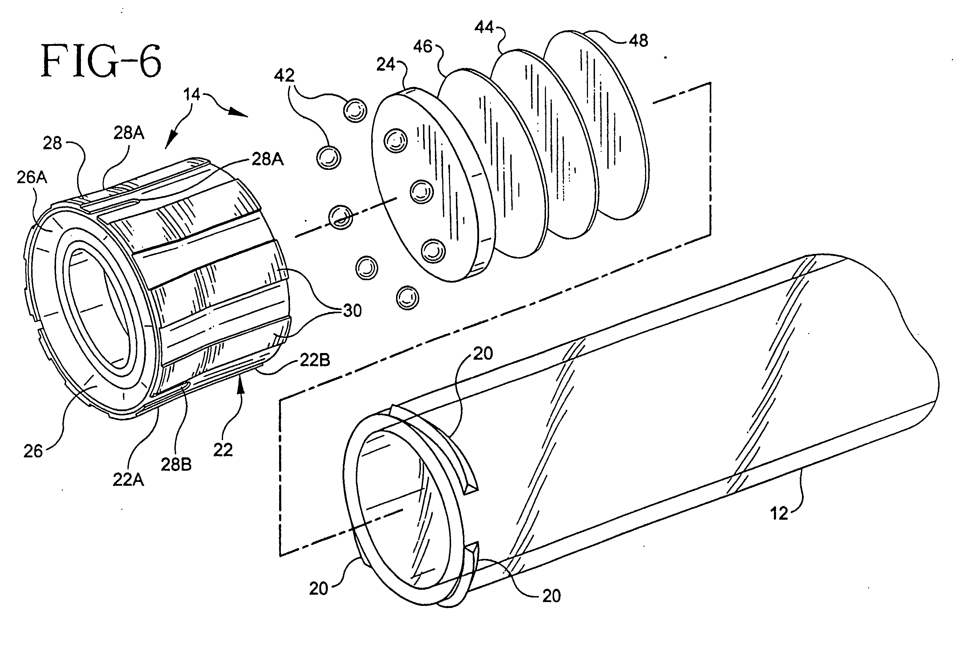

The container 12 is preferably transparent, and is preferably made from a material that is substantially gas-impermeable, such as glass or polyethylene terephthalate (PET). As shown in Fig. 6, the container includes an interrupted thread including three thread segments 20. Each thread segment has a first end portion adjoining the rim and a second end portion displaced spirally downwardly with respect to the first end portion. A gap separates each thread segment. The depth of the interrupted thread shown in the drawings is less than about 1,8 mm (0.070 inches)

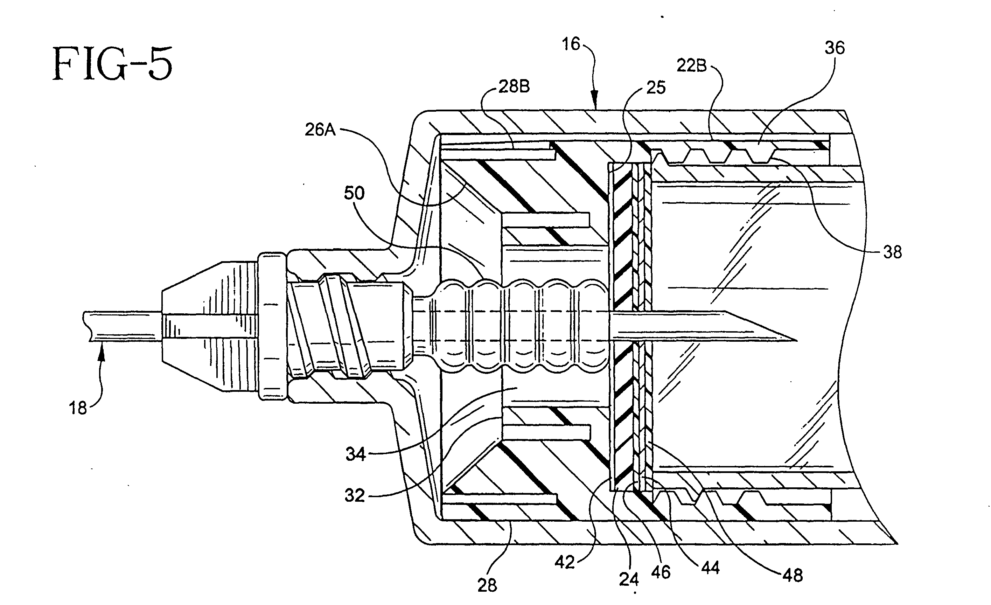

The closure assembly 14 is most clearly illustrated in Figs. 5 and 6. It includes a generally cylindrical cap 22 made of polypropylene or other semi-rigid polyolefin material and a resealable member 24. The resealable member is made from an elastomeric material such as polyurethane or, alternatively, butyl rubber, which is capable of resealing itself following piercing of the same by a needle and subsequent needle withdrawal.

The cap 22 includes a top portion 22A and a bottom portion 22B. An annular shoulder 25 is defined by the bottom surface of the top portion of the cap. The top portion of the cap further includes an annular, substantially rigid body portion 26 surrounded by an annular deflectable portion 28. The deflectable portion 28 is comprised of a plurality of arcuate walls 28A separated by slots 28B extending downwardly from the upper rim of the deflectable portion. The outer surface of the cap includes a plurality of elongate, radially extending protrusions 30. These protrusions facilitate the handling of the cap and assembly 10. They also provide contact surfaces in the event the assembly is dropped. Force is thereby imparted to the deflectable portion 28 of the cap.

The body portion 26 of the upper portion of the cap includes a chamfered upper surface 26A, which may include indicia such as opening and closing instructions. An annular wall 32 is positioned inwardly of the body portion 26. The inner surface of this annular wall 32 defines an axial opening 34 extending through the upper portion of the cap.

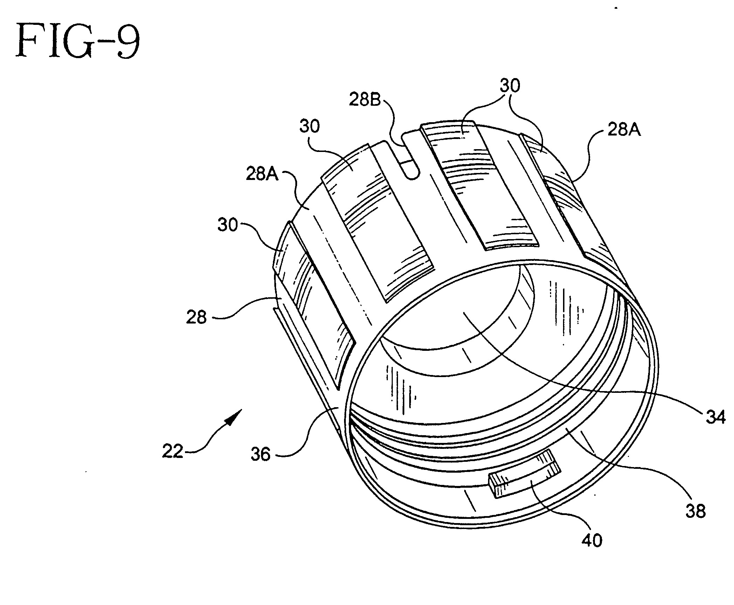

The bottom portion 22B of the cap 22 is defined by a substantially cylindrical wall 36 including a plurality of internal threads 38. In the embodiment shown herein, three spiral threads are provided, each having a thread lead adjoining a locating tab 40. (See Fig. 9). The locating tabs extend axially towards the bottom of the cap. The width of each tab is smaller than the width of each gap which separates the external thread segments 20 of the container 12. Each thread 38 extends spirally for 180° or less, preferably about 120-140°. This allows the cap 22 to be mounted to or removed from the container by turning it 180° or less. Such a task does not ordinarily require a two-handed operation. The cap can alternatively be snapped on the container. This feature facilitates the manufacturing process. The locating tabs facilitate rethreading the cap onto the container subsequent to its initial removal.

The resealable member 24 is shown in the drawings as a flat disc. This particular configuration is not critical, though a relatively thin center portion does facilitate needle penetration. The member 24 is secured to the shoulder 25, thereby closing the opening 34 through the upper portion of the cap 22. Hot melt adhesive 42 or other suitable adhesive may be used to permanently secure the resealable member to the cap.

A gas barrier member 44 is secured to the resealable member 24 by a tie layer 46 of adhesive material or a material such as PET. The gas barrier member is also bonded to the top portion of the container, and preferably the rim, by a seal layer 48 which may also be PET. (See Figs. 5 and 6). A seal layer which allows induction sealing is preferred. It should be understood that the drawings of the resealable member, gas barrier member and other sealing members are not to scale. The gas barrier member is preferably a metal foil, such as aluminum foil, having a thickness of about 0.001 inch. The tie and seal layers 46, 48 may be coatings on the gas barrier member.

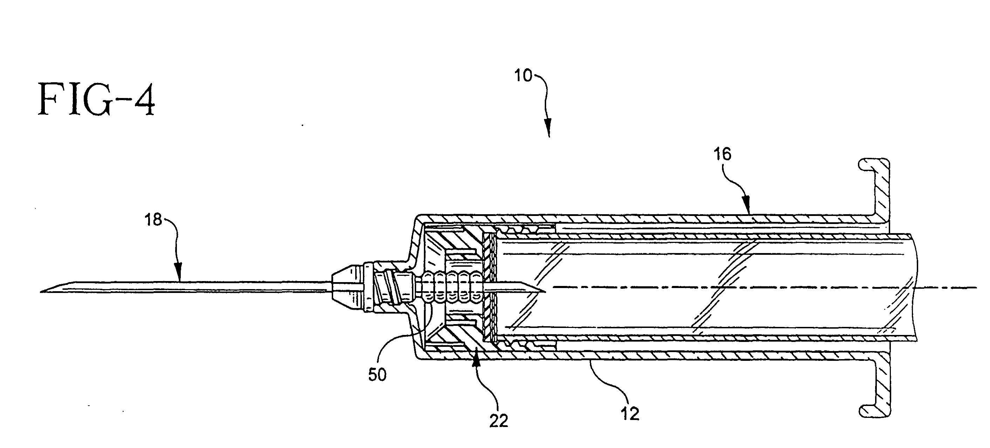

The assembly 10 as described above may be used with a holder 16 as shown in the drawings or other holders known to the art. As shown in Fig. 4, the assembly 10 is used to collect blood or other body fluid when inserted within the holder such that the rear end of the double-ended needle assembly 18 extends through the resealable member 24 and gas barrier member 44. The figures show a sheath 50 which is displaced upon insertion of the assembly 10 into the holder. The use of such a sheath is not required.

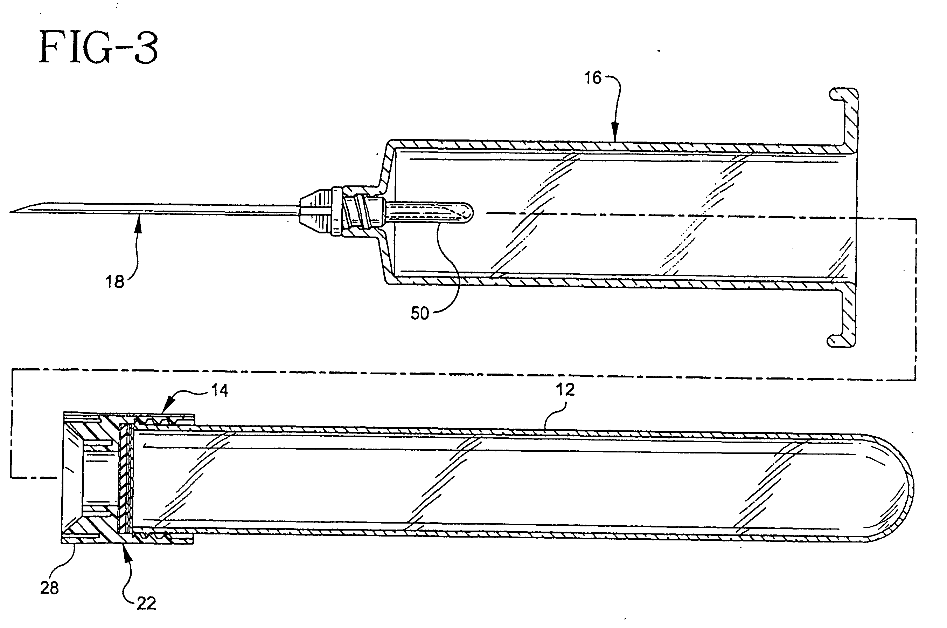

The assembly 10 is provided to users in the form shown in Figs. 1 and 3. The container 12 is ordinarily at least partially evacuated. As discussed above, the assembly 10 is designed to maintain a desired vacuum for a considerable length of time. The product should accordingly have a satisfactory shelf life.

Once the fluid collection procedure is completed, the assembly 10 is withdrawn from the holder 16. Though the closure assembly 14 is no longer gas-impermeable following such withdrawal, liquid impermeability is maintained by the resealable member. The assembly 10 can then be transported to an area where the fluid content of the container can be analyzed.

Removal of the closure assembly 14 is a relatively simple procedure which can be accomplished with one hand. The cap 22 is rotated about 120-140° in accordance with the preferred embodiment of the invention, and is then moved away from the open end of the container. The contents of the container can then be accessed. The closure assembly can be secured again to the open end of the container by first placing the cap over this end and then rotating it. The locating tabs 40 greatly facilitate the engagement of the container threads 20 and the cap threads 38.

Because the seal between the gas barrier member 44 and the container is relatively weak, this element preferably suffers no damage as the cap is turned and ultimately removed. The cap, resealable member and gas barrier member are accordingly removed as a unit and reapplied as a unit. The gas barrier member, being intact, forms a liquid-tight seal with the top rim of the container when the closure assembly is reapplied.

While the invention has been described with respect to the preferred embodiment thereof, it will be appreciated that alternative approaches may be taken with respect to the structures of the cap, sealing elements, and container. The threads employed for engaging the cap and container could, for example, be replaced by other engagement structures such as a bayonet locking arrangement (not shown).

## Claims

1. An assembly for collecting blood or other body fluids, comprising:
an elongate, generally cylindrical container (12) having an open end and a closed end;
a closure assembly (14) removable mounted to said open end of said container, said closure assembly including a cap (22) having a top and bottom, a resealable member (24) mounted to said cap (22), and an opening (34) in said cap, said resealable member being aligned with said opening;
**characterised in that**
said cap includes a deflectable portion (28) at the top of said cap and defining an outer surface of said cap.

2. An assembly as described in Claim 1, wherein said deflectable portion (28) is comprised of a plurality of deflectable members (28A) that define a generally cylindrical outer wall of said cap (22).

3. An assembly as described in Claim 2, including a plurality of axially extending slots (28B) defined within said generally cylindrical outer wall and separating said deflectable members (28A) and said cap (22) including a generally cylindrical body portion (26), said generally cylindrical wall being separated from said body portion (26) by a substantially annular slot.

4. An assembly as described in Claims 1 or 2, wherein said deflectable portion (28) is an integral part of said cap.

5. An assembly as described in one of Claims 1-4 including a tube holder (16) comprising a housing and a needle (18) coupled to said housing, said container (12) being at least partially insertable into said housing such that said needle (18) penetrates said resealable member (24) and said deflectable portion (28) of said cap (22) engages a wall of said housing, thereby frictionally maintaining said container in a selected position within said housing.

6. An assembly as described in one of Claims 1-5, including a gas barrier member (44) bonded to said open end of said container (12), said gas barrier member also being bonded to said resealable member (24), said resealable member being bonded to said cap (22), the bond between said gas barrier member (44) and said container (12) being weaker than the bond between said gas barrier member and said resealable member (24).

## Patentansprüche

1. Vorrichtung zum Sammeln von Blut oder anderen Körperflüssigkeiten, mit:
einem langgestreckten, im wesentlichen zylindrischen Behälter (12) mit einem offenen Ende und einem geschlossenen Ende,
einer Schließvorrichtung (14), die abnehmbar an dem offenen Ende des Behälters angebracht ist und die eine Kappe (22) mit einem oberen Ende und einem unteren Ende, ein an der Kappe (22) angebrachtes wiederabdichtendes Element (24) und eine Öffnung (34) in der Kappe aufweist, wobei das wiederabdichtende Element mit der Öffnung ausgerichtet ist;
**dadurch gekennzeichnet, dass**
die Kappe einen die Außenfläche der Kappe bildenden biegbaren Teil (28) am oberen Ende der Kappe aufweist.

2. Vorrichtung nach Anspruch 1, bei der der biegbare Teil (28) mehrere biegbare Elemente (28A) aufweist, die eine im wesentlichen zylindrische Außenwand der Kappe (22) bilden.

3. Vorrichtung nach Anspruch 2, mit mehreren axial verlaufenden Schlitzen (28B), die in der im wesentlichen zylindrischen Außenwand ausgebildet sind und die biegbaren Elemente (28A) und die Kappe (22), die einen im wesentlichen zylindrischen Körperteil (26) aufweist, voneinander trennen, wobei die im wesentlichen zylindrische Wand durch einen im wesentlichen ringförmigen Schlitz von dem Körperteil (26) getrennt ist.

4. Vorrichtung nach Anspruch 1 oder 2, bei der der biegbare Teil (28) integraler Bestandteil der Kappe ist.

5. Vorrichtung nach einem der Ansprüche 1 bis 4, mit einem Röhrchenhalter (16), der ein Gehäuse und eine mit dem Gehäuse verbundene Nadel (18) aufweist, wobei der Behälter (12) mindestens teilweise in das Gehäuse einsetzbar, so dass die Nadel (18) in das wiederabdichtende Element (24) eindringt und der biegbare Teil (28) der Kappe (22) an einer Wand des Gehäuses angreift und dadurch den Behälter in dem Gehäuse reibend in einer ausgewählten Position hält.

6. Vorrichtung nach einem der Ansprüche 1 bis 5, mit einem mit dem offenen Ende des Behälters (12) verbondeten Gassperrelement (44), das auch mit dem wiederabdichtenden Element (24) verbondet ist, welches mit der Kappe (22) verbondet ist, wobei die Verbondung zwischen dem Gassperrelement (44) und dem Behälter (12) schwächer ist als die Verbondung zwischen dem Gassperrelement und dem wiederabdichtenden Element (24).

## Revendications

1. Ensemble destiné à recueillir du sang ou d'autres liquides corporels comprenant :
un récipient (12) généralement cylindrique, allongé présentant une extrémité ouverte et une extrémité fermée ;
un ensemble de fermeture (14) monté de manière amovible sur ladite extrémité ouverte dudit récipient, ledit ensemble de fermeture comprenant un capuchon (22) ayant un haut et un bas, un organe apte à être réétanchéifié (24) monté sur ledit capuchon (22), et une ouverture (34) dans ledit capuchon, ledit organe apte à être réétanchéifié étant aligné avec ladite ouverture ;
**caractérisé en ce que**
ledit capuchon comprend, sur le haut dudit capuchon, une partie pouvant dévier (28) et définissant une surface extérieure dudit capuchon.

2. Ensemble selon la revendication 1, dans lequel ladite partie pouvant dévier comprend une pluralité d'éléments pouvant dévier (28A) qui définissent une paroi extérieure généralement cylindrique dudit capuchon (22).

3. Ensemble selon la revendication 2, comprenant une pluralité de fentes s'étendant axialement (28B) définies dans ladite paroi extérieure généralement cylindrique et séparant lesdits éléments pouvant dévier (28A) et ledit capuchon (22) comprenant une partie de corps généralement cylindrique (26), ladite paroi généralement cylindrique étant séparée de ladite partie de corps (26) par une fente essentiellement annulaire.

4. Ensemble selon les revendications 1 ou 2, dans lequel ladite partie pouvant dévier (28) fait partie intégrante dudit capuchon.

5. Ensemble selon l'une quelconque des revendications 1 à 4, comprenant un support de tube (16) comprenant un logement et une aiguille (18) couplée audit logement, ledit récipient (12) pouvant être inséré au moins partiellement à l'intérieur dudit logement, telle que ladite aiguille pénètre ledit organe apte à être réétanchéifié (24) et ladite partie pouvant dévier (28) dudit capuchon (22) vient en prise avec une paroi dudit logement, et maintenant de cette façon, par frottement, ledit récipient dans une position choisie à l'intérieur dudit logement.

6. Ensemble selon l'une quelconque des revendications 1 - 5, comprenant un élément formant barrière de gaz (44) relié à ladite extrémité ouverte dudit récipient (12), ledit élément formant barrière de gaz étant aussi relié audit organe apte à être réétanchéifié (24), ledit organe apte à être réétanchéifié étant relié audit capuchon (22), la liaison entre ledit élément formant barrière de gaz (44) et ledit récipient (12) étant plus faible que la liaison entre élément formant barrière de gaz et ledit organe apte à être réétanchéifié (24).
